# EUROPEAN PATENT SPECIFICATION

(11) **EP 2 816 354 B1**
(45) Date of publication and mention of the grant of the patent: **28.09.2016**
(21) Application number: 13172634.1
(22) Date of filing: 19.06.2013
(51) Int. Cl.: G01N 33/564, G01N 33/68

(54) **Biomarker for complex regional pain syndrome (CRPS)**
Biomarker für die Krankheit "complex regional pain syndrome" (CRPS)
Biomarqueur pour la maladie "complex regional pain syndrome" (CRPS)

(43) Date of publication of application: 24.12.2014
(73) Proprietor: Medizinische Hochschule Hannover, 30625 Hannover (DE)
(72) Inventor: Baerlecken, Niklas Thomas, Dr.med., 30171 Hannover (DE); Witte, Torsten, Prof.Dr.med., 30559 Hannover (DE); Bernateck, Michael, Dr.med., 30519 Hannover (DE)
(74) Representative: Kröncke, Rolf

(56) References cited:
- EP-A1- 2 199 305
- US-A1- 2005 097 626
- KOHR D ET AL: "Autoantibodies in complex regional pain syndrome bind to a differentiation-dependent neuronal surface autoantigen", PAIN, ELSEVIER SCIENCE PUBLISHERS, AMSTERDAM, NL, vol. 143, no. 3, 1 June 2009 (2009-06-01), pages 246-251, XP026851890, ISSN: 0304-3959 [retrieved on 2009-04-16]

## Description

The present invention relates in a first aspect to a method for diagnosing the presence or the risk of development or the therapy control of Complex Regional Pain Syndrome (CRPS) and other inflammatory diseases including the joints, e.g. spondyloarhtritis and rheumatoid arthritis. The method comprises the steps of analyzing a sample from an individual to be tested for the presence of autoantibodies against the inhibitor of growth (ING) family proteins, in particular, the p29ING4 protein whereby the presence of said autoantibodies against ING, in particular, p29ING4 is indicative for the presence or the risk of development CRPS. In another aspect, the present invention relates to the use of a diagnostic kit in the method according to the present invention. Further, the present application relates to the use of the ING proteins, in particular, the p29ING4 protein or immunoreactive peptides thereof in the diagnosis, risk assessment or therapy control of CRPS or developing CRPS or other inflammatory diseases involving the joints, in particular, in subjects undergoing scheduled surgery of hands and feet.

### Prior art

CRPS is a syndrome characterized by a continuing (spontaneous and/or evoked) regional pain that is seemingly disproportionate in time or degree to the usual cause of any known trauma or other lesion. The pain is regional (not in a specific nerve territory or dermatome) and usually has a distal predominance of abnormal sensory, motor, sudomotor, vasomotor, and/or trophic findings. The syndrome shows variable progression over time.

Various clinical diagnostic criteria for CRPS exist. A recent publication of Harden, R.N. et al., Pain Med., 2013, doi:10.1111/PME.12033 provides an overview thereof. The clinical diagnostic criteria include continuing pain which is disproportionate to any inciting event, at least one symptom of different functions and categories as outlined therein including sensory, vasomotor, sudomotor/edema, motor/throphic symptoms. Remarkably, the clinical diagnostics criteria for CRPS include the criteria: *"there is no other diagnosis that better explains the signs and symptoms".* Thus, it is clear, that at present diagnosis of CRPS is based on excluding other diseases and conditions, i.e. a negative diagnosis only.

CRPS can be differentiated into various subtypes, namely, CRPS I, old name "reflex sympathetic dystrophy"; CRPS II, old name "causalgia"; and CRPS-NOS (not otherwise specified) including all individuals which were not categorized in I or II and which partially meets CRPS criteria but not better explained by any other condition. At present, the current diagnostic guidelines distinguish between CRPS without (CRPS I) or with obvious nerve lesions (CRPS II). The main symptom of CRPS is severe neuropathic pain located deep and distally accented in the involved limb with concomitant hyperalgesia, one third of patients also suffer from allodynia (brush-evoked pain). Autonomic and trophic disturbances, such as edema and swelling of the limb, skin changes, altered growth of hair and nails, and increased or decreased sweating can additionally be observed. Most patients have a weakness of the effected limb, with tremor, myoclonus, and focal dystonia frequently present.

Typically, CRPS I often occurs in the hands or feet after a minor injury, while CRPS II is caused by injury to a specific nerve. It is thought that CRPS is the result from damages to the nervous system, including the nerves that control the blood vessels and sweat glands.

As CRPS is a clinical diagnosis, the characteristic clinical symptoms have to be recognized and differentiated from ordinary posttraumatic or post operative findings and by the existence of other conditions or diseases. As mentioned above, at present diagnosis is based on the fact that other diseases can be excluded and *"there is no other diagnosis that better explains the signs and symptoms".* During the early phase following trauma this discrimination is difficult because of similarities between CRPS and injury-related symptoms. Thus, the first diagnosis of CRPS is made in many cases too late, months or even years after the onset of symptoms in some patients. Evidences suggest that the earlier specific treatment is started, the more successful it is.

When looking into the literature, it is clear that diagnosing CRPS is difficult, but early diagnosis is very important. Typically, the doctor will take a medical history and do a physical examination. Other tests may include a method to show temperature differences and a lack of blood supply in the affected limb (thermography), as well as bone scans, nerve conduction studies and x-rays. For example, clinical diagnosis of CRPS can be assisted and specified by 3-phase-bone-scintigraphy (TPBS) which is currently the gold standard of the imaging procedures. An accelerated blood flow into the affected limb combined with an increased periarticular activity during the blood pool phase and the delayed static phase are classical findings of TPBS, but the sensitivity (65 %) and specificity (50 %) of TPBS for the diagnosis of CRPS I is variable throughout the literature. Aside time and effort of TPBS sensitivity and specificity of TPBS significantly decreased already three to six months after symptom onset.

Thus, it is clear, that diagnosis is complicated by the fact that some patients improve without treatment and a delay in diagnosis and/or treatment for the syndrome can result in severe physical and psychological problems.

CRPS is a frequent complication after trauma or operation with a prevalence of about 30/100.000. Prospective studies in patients with distal radial fracture revealed a more than 20 % incident of this complication. Hence, CRPS has a relevant social impact, because it frequently causes severe long standing disability in the afflicted patients. Of note, the incidence of rheumatoid arthritis is 30/100.000, while for MS 4/100.000.

Recently, autoimmunity against the β-2 adrenergic receptor and muscarinic-2 receptor in CRPS was described, Kohr D., et al., 2011, Pain, 152, 2690-2700. Therein, identification of functionally active autoantibodies in serum samples from CRPS patients are described. In addition, antibodies directed against antigens of the autonomic nervous system, shown by reactivity of patients are sympathetic and myentheriplexus neurons as well as cholinergic neuroplastome cells have been detected as well. The origin of the antibodies was not known and their specificity was not established. In addition, it is not known whether the antibodies were already present before trauma. In WO 2010/069570 peptides against autoantibodies associated with CRPS and use of these peptides have been described, based on the finding of autoantibodies against the above-cited receptor molecules.

Finally, it has been speculated that micro-RNAs (miRNAs) modulation may be used as diagnostic tool also in CRPS. Orlova IA., et al., Journal of Translational Medicine, 2011, 9:195 described differences in miRNAs profiles that may be useful in patients stratification and have utility as potential bi-omarkers for pain.

Spondyloarthritis (Spa) also known as spondyloarthropaty or spondylitis identifies a group of diseases primarily affecting the spine and other joints. This group of diseases is also identified as seronegative spondyloarthritis. The term "seronegative" refers to the fact that usually no rheumatoid factors are present in the blood. The group of Spa can be divided into Ankylosing Spondylitis (AS), reactive arthritis and its special manifestation, Reiter Syndrome, and into psoriatic arthritis (PsA), enteropatic arthritis and undifferentiated spondyloarthritis.

Also for Rheumatoid arthritis (RA), another example of an inflammatory joint disease, diagnosis and management of said disease is important. RA is a chronic, systemic inflammatory disorder that may affects many tissues in organs, but principally attacks flexible joints. Until today RA diagnosis is based mainly on Y-rays imaging only.

ING4 inhibitor of growth family, member 4, (ING4), is a gene encoding a tumor protein that contains a PHD-finger, which is a common motive in proteins involved in chromatin remodelling. This protein combines TP53 and EP300/P300, a component of the histone acetyl transferase Complex, suggesting its involvement in TP53 dependent on regulatory pathway. The ING4 gene belongs to the ING (inhibitor of growth) tumor suppressor. An overview of the ING family is given in Gunduz, E., et al., J. Hard tissue biology, 2008, 17(1), 1-10. Thr ING family comprises at least 7 members, ING1 a, b, and c, ING 2 to 5.

Involvement of one of the ING family member ING4 in various types of tumors and cancers as well its use as a marker in tumor diagnosis and therapy has been discussed in the literature. ING4 is also known as p29ING4. Of note, ING4 has not been discussed with respect to pain so far.
Kohr D. et al, PAIN, 2009, 143, 246-251 describe autoantibodies in CRPS binding to a differentiation-dependent neuronal surface autoantigen. EP 2 199 305 A1 relates to peptides against autoantibodies associated with CPRS and use of these peptides. Further, US 2005/0097626 A1 relates to the tumor suppressor gene p28ING5 is general without any association to autoimmune diseases.

In view of the above, in particular, in view of the clinical criteria for diagnosing CRPS, in particular, CRPS 1 including the criteria of *"there is no other diagnosis that better explains the signs and symptoms of the individual"* it is clear that there is a need for providing a diagnostic tool and a method for supporting diagnosis and assessing the risk of developing CRPS including CRPS I and CRPS II, as well as a tool and method for therapy control thereof.

Further, there is a need for providing a diagnostic tool and a method for supporting diagnosis and assessing the risk of developing other inflammatory diseases involving the joints, including RA and Spa, like AS and undifferentiated spondyloarthritis.

In addition, this tool, e.g. the kit, or a method should preferably allow to differentiate between CRPS and other types of inflammatory diseases, in particular osteoarthritis, or other non-inflammatory joint diseases.

### Brief description of the present invention

The present invention aims in providing a method for diagnosing the presence or the risk of development, or for therapy control of Complex Regional Pain Syndrome (CRPS), preferably of CRPS I, or other inflammatory diseases involving the joints in a subject. That is, the present inventors recognized that subjects or individuals suffering from CRPS have at least autoantibodies directed against ING family members, in particular, the molecule p29ING4, that is, the gene product of ING4. It has been recognized that the presence of autoantibodies against ING, like p29ING4, are indicative for CRPS or sensitive for the risk of development CRPS or important for treatment control of CRPS. In particular, earlier diagnosis of CRPS is possible making the laborious diagnosis redundant for excluding other diseases. Thus, a first embodiment relates to a method for diagnosing the presence or the risk of development, or for the therapy control of inflammatory disease involving the joint including RA and Spa, in particular for diagnosing the presence or the risk of development, or for the therapy control of CRPS, preferably of CRPS I, in a subject comprising on a biological sample obtained from the subject, analysing the sample from the subject for the presence of autoantibodies against a ING family member, in particular, p29ING4,
- whereby the presence of autoantibodies against the ING family member, in particular, p29ING4 is indicative of the presence or the risk of development, or for the treatment control of the inflammatory disease involving joints, in particular, of CRPS.

Preferably, IgA and IgG autoantibodies are detected. In another preferred embodiment, the subject is a human and the autoantibodies are human autoantibodies.

Another embodiment of the present invention relates to the use of a diagnostic kit in a method according to the present invention for diagnosing the presence or risk of a development as well as for treatment control of the inflammatory disease involving joints, in particular, of CRPS in a subject comprising means of determining autoantibodies against an ING gene product of the ING family, in particular, the P29ING4 protein or immunological peptides derived therefrom in a biological sample of subject to be tested and instructions how to use said test kit. Preferably, said test kit is an ELISA assay.

### Brief description of the drawings

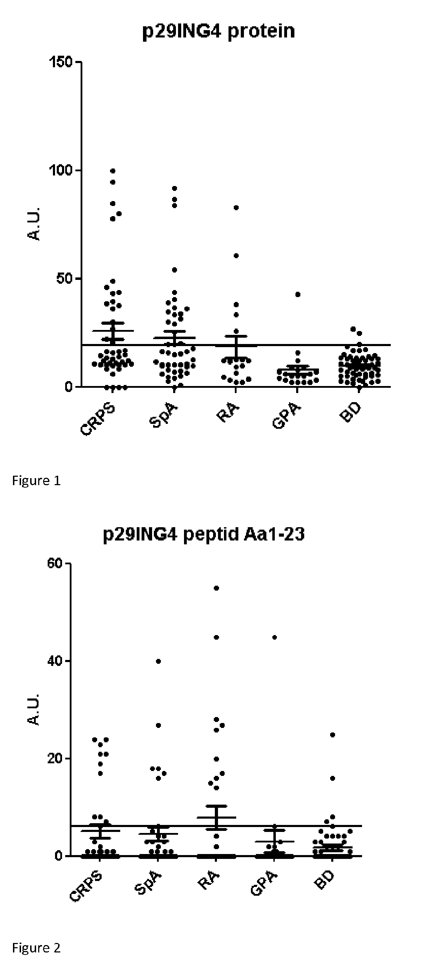

Figure 1: The figure shows the arbitrary units of the antibodies binding to p29ING4 protein in complex regional pain syndrome (CRPS, n=46), spondy-loarthritis (SpA, n=46), rheumatoid arthritis (RA, n=36), Granulomatosis with Polyangitis (GPA, n=40) and blood donors (BD, n=56).
Figure 2: The figure shows the arbitrary units of the antibodies binding to p29ING4 peptide aa1-23 in complex regional pain syndrome (CRPS, n=46), spondyloarthritis (SpA, n=46), rheumatoid arthritis (RA, n=36), Granulomatosis with Polyangitis (GPA, n=40) and blood donors (BD, n=56).

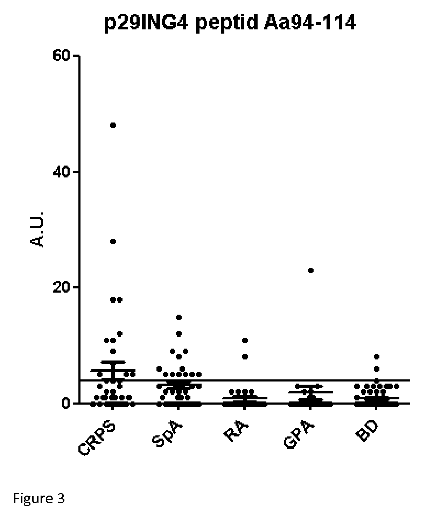

Figure 3: The figure shows the arbitrary units of the antibodies binding to p29ING4 peptide aa94-114 in complex regional pain syndrome (CRPS, n=46), spondyloarthritis (SpA, n=46), rheumatoid arthritis (RA, n=36), Granulomatosis with Polyangitis (GPA, n=40) and blood donors (BD, n=56).

### Detailed description of the present invention

In a first aspect, the present invention relates to a method for diagnosing the presence or the risk of development, or for the therapy control of an inflammatory disease involving joints including RA and Spa, in particular, of CRPS, preferably of CRPS1, in a subject comprising
on a biological sample obtained from the subject
analysing the sample for the presence of autoantibodies against an ING gene product, in particular, against p29ING4,
whereby the presence of autoantibodies against the ING gene product, in particular, p29ING4 is indicative of the presence or the risk of development, or for the therapy control of the inflammatory disease involving joints including RA and Spa, in particular, of CRPS.

That is, the present invention is based on the observation of present inventor that subjects afflicted with CRPS or having risk of developing CRPS as well as for therapeutic control of CRPS have autoantibodies against the ING gene product, in particular, p29ING4. The same holds true for subjects afflicted with other types of inflammatory diseases involving joint inflammation including RA and Spa.

For example, in case of Spa including AS and undifferentiated spondy-loarthritis, the method according to the present invention may be combined with the method as described in WO2012/022459 including the detection of autoantibodies directed agains CD74 and/or IKBKB.

With respect to the term "autoantibody" or "autoantibodies" is meant an autoantibody that is directed against one or more of the subjects own proteins.

As used herein, the term "ING gene product" refers to a peptide, e.g. the polypeptide or the protein, of the gene encoding an ING family member including the ING1a, ING1b, ING1c, ING2, ING3, ING4, and ING5 gene.

P29ING4 is the gen product of the ING4 gene. The gene product is expressed by a variety of cells. The present inventors demonstrate that the gene product of ING genes, including P29ING4 represent entities to which autoantibodies can be found in subjects afflicted with CPRS or other types of inflammatory joint diseases including RA and Spa. Hence, determining the presence of autoantibodies against CRPS or other types of inflammatory diseases involving the joints including RA and Spa are indicative of the presence or the risk of development, or for the therapy control of CPRS or other types of inflammatory diseases involving the joints including RA and Spa in a subject. As demonstrated in the examples, determining autoantibodies against the gene product of ING genes, in particular, against P29ING4 allow to identify individuals suffering from CPRS or other types of inflammatory joint diseases including RA and Spa.

In a preferred embodiment, the methods allow to determine CPRS I. In another advantageous embodiment, the presence of autoantibodies against p29ING4 is determined.

The analysis and determination of the presence of autoantibodies against the ING gene product, in particular, against p29ING4 allows for the first time a high-sensitive diagnostics of CRPS and a classification of CRPS from other diseases and conditions having similar symptoms.

The diagnostic method is particularly relevant for diagnosis in humans. The diagnosis may be relevant not only in case of detecting the presence of CRPS but also for assessing and diagnosing the risk of development of CRPS. This might be relevant for example in case of any type of scheduled surgery of hands and feet to be conducted in said individuals. For example, in case of a necessary surgery, the individual may be tested in advance for the risk of development CPRS based on the presence of autoantibodies against an ING gene product, in particular, against p29ING4 and in case of a positive result, prophylactic arrangements could be performed or the monitoring could be tightened.

Moreover, detecting autoantibodies against an ING gene product, in particular, against p29ING4 can be used for treatment control in case of manifest CRPS or other types of inflammatory diseases involving joints including RA and Spa. That is, the presence of autoantibodies against an ING gene product, for example, against p29ING4, in particular, development of new autoantibodies and cells producing the same during therapy may be controlled accordingly.

As used herein, the terms "individual", "patient" and "subject" are used interchangeably and refer to patients and subjects of humans or other mammals and include any individual it is desired to examine or treat using the methods of the present invention. However, it will be understood that "patient" does not imply that symptoms are present.

The term "biological sample" as used herein refers to a sample obtained before from the subject that may be extracted, untreated, treated, isolated or concentrated therefrom. Of note, the method according to the present invention does not include the step of taking the sample from the subject. Rather, the sample is an isolated sample which may be processed further. Suitably, the biological sample is selected from any part of the patient's body, including, but not limited to hair, skin, nails, tissues or body fluids, such as saliva, synovia and blood as well as liquor.

Throughout the specification, unless the context requires otherwise, the word "comprise" will be understood to imply the inclusion of a stated step or element or group of steps or elements but not the exclusion of any other step or element or groups of steps of elements.

Further, the term "comprise" or "comprising" as well as "obtain" or "obtaining" include the embodiment of "consist" or "consisting of".

With the term "obtained from" is meant that a sample such as for example, serum is isolated from or derived from a particular source of the subject including obtaining the same physically from the subject. The biological sample is obtained in advance and the method according to the present invention includes the use of said isolated biological sample previously isolated from the subject to be tested and physically determining the presence or absence of the autoantibodies as described herein.

As used herein, the terms "a", "an" and "the" mean "one or more" are used in this application, including the claims, unless otherwise indicated.

Unless defined otherwise, all technical and scientific terms used herein have the same meaning as commonly understood to one of the ordinary skilled in the art to which the present disclosed subject matter belongs.

The terms "diagnosing" and "diagnosis" as used herein refers to methods by which a skilled artisan can estimate and even determine whether or not a subject suffering from a given disease, disorder or condition. The skilled artisan makes the diagnosis on the basis of one or more diagnostic indicators, namely autoantibodies, the amount (including presence or absence) of which is indicator for the presence, severity, or absence of the condition.

Hence, "making a diagnosis" or "diagnosing", as used herein, may further include making a prognosis which can provide for predicting a clinical outcome, selecting an appropriate treatment, or monitoring a current treatment and potentially change in the treatment based on the measure of a diagnostic autoantibody.

The term "determining" or "analyzing" as used herein refers to assessing the presence, absence, quantity, level or amount of the respective autoantibodies within the subject derived sample, including qualitative or quantitative concentration levels of said substances otherwise evaluating the values or categorization of a subject clinical parameter. In particular, said terms include the physical detection or analysis, respectively. It has been determined by the inventors that autoantibodies against an ING gene product, in particular, against p29ING4 can be correlated with the presence or the risk of developing CRPS, in particular, CRPS I, or other types of inflammatory diseases involving joints including RA and Spa.

In addition, in some embodiments of the presently disclosed subject matter, multiple determinations of autoantibodies over time can be made to facilitate diagnosis and/or prognosis.

As identified above, it is preferred that the isolated biological sample is selected from blood, tissue or fluid preferably selected from hair, skin, nails, saliva, synovial, liquor and blood. It is particularly preferred, that the biological sample is an isolated body fluid, preferably blood, in particular, serum.

Typically, the methods according to the present invention are in vitro methods.

The methods according to the present invention allow the diagnosis of the presence or risk of development of CRPS or other types of inflammatory joint diseases including RA and Spa, as stated above. Moreover, the method according to the present invention allows for the stratification of the therapeutic regimen of a subject afflicted with CRPS or other types of inflammatory joint diseases including RA and Spa, or being at risk of developing CRPS or other types of inflammatory joint diseases including RA and Spa. Moreover, the methods according to the present invention allows to identify the stratification of the disease, in particular, the activity of the disease and the subject afflicted with CRPS or other types of inflammatory joint diseases including RA and Spa.

The skilled person is well aware of useful immunodetection methods allowing analyzing the sample for the presence or absence of autoantibodies against an ING gene product, in particular, against p29ING4. For example, the biological sample obtained from the subject is contacted with an antigen, namely an ING gene product, e.g. with a p29ING4 oligo-, polypeptide or protein representing the autoantibody immunoreactive peptide, thus, allowing binding of the autoantibody to said peptide. In this connection, the terms polypeptide or protein which are used interchangeably herein, refer to a polymer of amino acids having a length of at least 50 aa. The term "oligopeptide" refers to a polymer of amino acids having a length of from 5 to 49 aa.

Contacting the chosen biological sample of the antigen under conditions effective and for a period of time sufficient to allow the formation of immune complexes, is generally a matter of adding the composition to the sample and incubating the mixture for a period of time along enough for the autoantibodies to form immune complexes with the antigens presented. Said antigen antibody mixture can be detected by known means and methods. That is, detection of immunocomplex formation of antigen autoantibody can be achieved through the application of numerous approaches. These methods are generally based upon the detection of a label or marker, such as any radioactive, fluorescent, biological or enzymatic tags or labels of standard use in the art. Of course, one may find additional advantages through the use of a secondary binding ligand such as a second antibody or a biotine/avidine (streptavidine) ligand binding arrangement as it is known in the art.

In some embodiments, the primary immune complex can be detected by a second binding ligand that has binding affinity for the antigen or the autoantibody presented in the sample, for example reactivity to the Fc region of the autoantibodies or having reactivity to a region of the antigen different to the binding region of the autoantibody. In these cases, the second binding ligand can be linked to a detectable label or marker molecule. The second binding ligand is itself often an antibody which may thus be termed a secondary antibody. Typically, the primary immune complexes are contacted with the labelled, secondary binding ligand or antibody, under conditions effective and for a period of time sufficient to allow the formation of secondary immune complexes. The secondary immune complexes are then generally washed to remove of any unbound labelled secondary antibodies or ligands, and the remaining label in the secondary immune complex is then detected.

The second binding ligand, such as an antibody, having binding activity for either the antigen or autoantibody, may also be used to bind to the primary immune complexes. The second binding ligand contains an enzyme capable of processing a substrate detectable to a product and, hence, amplify a signal over time. After washing, the secondary immune complexes are contacted with substrate, permitting detection.

Alternatively, comparative immunodetection may be used. The skilled person is well aware of suitable methods.

It is particularly preferred that the biological sample is a body fluid, preferably blood. In particular, the biological sample is serum of the subject to be diagnosed.

Further, in another embodiment, the methods according to the present invention allows for the stratification of the therapeutic regimen of a subject afflicted with CRPS, or being at risk of developing CRPS. That is, the present invention allows to identify the status of disease, in particular, the active state of the disease in a subject afflicted with CRPS.

The autoantibodies to be detected may be of the IgA and/or IgG type. That is, it is possible to determine the presence of IgA and/or IgG autoantibodies in the biological sample obtained from the subject. Although it is sufficient to detect only one type of antibodies, either IgA or IgG autoantibodies, it is preferred that both IgA and IgG autoantibodies against an ING gene product, in particular, against p29ING4 are detected.

In a particular preferred embodiment, the subject is a human and the autoantibodies are human autoantibodies.

In a further embodiment, the presently disclosed subject matter provides the use of test kits or diagnostic kits in a method according to the present invention. In particular, immunological kits for use in detecting autoantibodies in biological samples allowing diagnosis of CRPS, like CRPS I, or other types of inflammatory diseases involving joints including RA and Spa. That is, the present invention provides the use of a test kit in a method according to the present invention for diagnosing the presence or the risk of a development as well as for the therapy control of CRPS or other types of inflammatory diseases involving joints including RA and Spa, in a subject comprising means or determining autoantibodies against an ING gene product, in particular, against p29ING4 in a biological sample of a subject to be tested and instructions on how to use the test kit. In a preferred embodiment, said test kit is an ELISA.

Such kits can generally comprise one or more antigens, namely, oligo- or polypeptides of the ING gene product, in particular of p29ING4 that can immunoreact with the autoantibodies. Typically, the immunodetection kits will comprise in suitable container(s), one or more autoantibody immunoreactive peptide antigens derived from the ING gene product, in particular, from p29ING4. Said antigens useful in the presently claimed methods and test kits may be the full ING gene product, in particular, the p29ING4 protein or immune reactive peptides derived therefrom.

For example, the immune reactive peptide is at least one peptide selected from a peptide of Seq. ID. No. 1, Seq. ID. No.2, Seq. ID. No.3, Seq.ID.No.4, and Seq.ID.No.5. It is preferred that an immune reactive peptide comprising the peptide of Seq. ID. No. 1 is used. In case of CRPS, it is also favourable to use an immune reactive peptide comprising the sequence of Seq. ID. No. 5.

In certain embodiments, the antigen can be provided bound to a solid support, such as for example a column matrix or a well of a microtiter plate, a membrane, beads, dip sticks or the like. Alternatively, the support can be provided as a separate element of the kit.

That is, the use of the test kit according to the present invention in diagnosing CRPS or other types of inflammatory joint diseases including RA and Spa, includes beside the antigen a detection agent for the autoantibodies which may be an antibody, antibody fragment, etc. In addition, the kit may comprise more than one detection agent. If required, the kit further comprises substrate and further means for allowing reaction with an enzyme used as label for the detecting agent which may be an antibody.

The immunodetection agents of the kit can include detectable labels that are associated with or linked to the given detecting agent, in particular, the detecting antibody. Detectable labels that are associated with or attached to a secondary binding ligand are also contemplated. Detectable labels include dyes, illuminescent or fluorescent molecules, biotine, radiolabels or enzymes. Typical examples for suitable labels include commonly known fluorescent molecules, like rhodamine, fluorescein, green fluorescent protein or luciferase, or alkaline phosphatase and horseradish peroxidase as examples for suitable enzymes.

Optionally, the kits further comprise positive and negative controls for verifying the results obtained when using the kit. The components of the kits can be packaged either in aqueous medium or lyophilised form and, in addition, the kits comprise one or more containers allowing to conduct the detection. In addition, the test kit comprises instructions for use of the kit.

Moreover, the present invention relates to the use of an ING gene product, in particular, of p29ING4, immune reactive sequences or analogs thereof in the diagnosis, risk assessment or therapy control of CRPS or other types of inflammatory diseases involving joints including RA and Spa.

In this connection, the term "immune reactive peptides" refers to peptide fragments of an ING gene product, in particular, of p29ING4 enabling binding with the autoantibodies derived from the subject.

### Examples

The following example has been included to illustrate the present disclosed subject matter.

### Example 1

70 sera of patients having different types of different inflammatory and rheumatic diseases were screened via protein microarray technology. A new marker of CRPS has been identified which has been evaluated further for its frequency and association accordingly.

In protein array screening, 4 of 6 patients with CRPS showed antibodies against p29ING4, 1/6 antibodies against p29ING4 and p27ING3 and 1/6 against p28ING5.

In ELISA, an evaluation was effected with sera of 46 patients with CRPS, 32 blood donors, 46 patients with spondyloarthritis (SpA), 40 patients with granulomatosis with polyangitis (GPA) and 36 having rheumatoid arthritis (RA). The patients have been well characterized with regard to the disease activity, demographic data and treatment. The sera of the patients of the controls had been stored in a -20°C freezer until use.

### ELISA:

For each of the following ELISAs we tested different conditions and each component including the plates, blocking solutions and coating solutions was tested for unspecificity.

### Detection of autoantibodies against p29ING4:

In this ELISA, a recombinant full length protein of was used as the antigen. Its purity was >90% by SDS-PAGE and it was derived in an *Escherichia coli* host expression system (Source Biosciences Lifesciences, UK). For performing the ELISA tests, 96 well plates (Maxisorb, Nunc, Denmark) were coated with 1 µg human per well and phosphate saline buffer (PBS) over night. Then the plates were blocked with 300µl PBS and 1% fat reduced milk powder (Saliter, Obergünzburg, Germany) per well for 30 minutes at room temperature (RT). The plates were incubated with 100 µl diluted sera (1:100) in Tris-phophate buffer (TBS) with 1% fat reduced milk powder for 30 minutes at RT. After 30 minutes of incubation, the plates were washed 3 times with 300 µl 0.05% Tween-20 PBS (PBS-T) (Thermo Fisher Scientific, DK). Next, 100 µl of a secondary goat anti-human IgG (Fcy fragment specific) labelled with horse radish peroxidase (HRP) (Jackson ImmunoResearch Europe Ltd.) was added in a dilution of 2:10000 or 2 µl in 10 ml PBS with 1% fat reduced milk powder. Instead of secondary goat anti-human IgG (Fcy fragment specific) labelled with HRP (Jackson ImmunoResearch Europe Ltd.), a secondary goat anti-human IgA labelled with HRP was added in a dilution of 3:10000 or 3µl in 10ml PBS with 1% fat reduced milk powder for the detection of IgA autoantibodies. The plates were incubated for 30 min at room temperature and washed 3 times with PBS-T. The color reaction was performed with TMB (Thermo Fisher Scientific, DK) for up to 30 min according to the manufacturer's instructions and the ODs were read at 450 nm in an ELISA reader. As a standard a serum of a patient with a high OD was used. The concentration of IgG antibodies against in this serum was defined as 100 U/ml.

### Detection of autoantibodies binding to peptides of p29ING4:

We used the following peptides (Biomatik, Wilmington, Delaware, USA): Amino acid sequence
(Aa)1-23 MAAGMYLEHYLDSIENLPFELQR (Seq. ID. No.1)
Aa24-44 FQLMRDLDQRTEDLKAEIDK (Seq. ID. No.2)
Aa45-72 LATEYMSSARSLSSEEKLALLKQIQEAYG (Seq. ID. No.3)
Aa71-93 AYGKCKEFGDDKVQLAMQTYEMV (Seq. ID. No.4)
Aa94-114 DKHIRRLDTDLARFEADLKEK (Seq. ID. No.5)

For performing the ELISA tests, 96 well plates (Maxisorb, Nunc, Denmark) were coated with 1 µg human synthetic peptide of p29ING4 per well and phosphate saline buffer (PBS) over night. Then the plates were blocked with 300µl PBS and 0.2% casein (Thermo Fisher Scientific, DK) per well for 30 minutes at room temperature (RT). The plates were incubated with 100 µl diluted sera (1:100) in PBS 0.2% Casein for 30 minutes at RT. After 30 minutes of incubation, the plates were washed 3 times with 300 µl 0.05% Tween-20 PBS (PBS-T) (Thermo Fisher Scientific, DK). Next, 100 µl of a secondary peroxidase-goat anti-human IgG Fcy specific labelled with horse radish peroxidase (HRP) (Jackson ImmunoResearch Europe Ltd.) was added in a dilution of 2:10000 or 2 µl in 10 ml 1% casein PBS. The plates were incubated for 30 min at room temperature and washed 3 times with PBS-T. The color reaction was performed with TMB (Thermo Fisher Scientific, DK) for up to 30 min according to the manufacturer's instructions and the ODs were read at 450 nm in an ELISA reader. As a standard a serum of a patient with a high OD was used. The concentration of IgG antibodies against each peptide in this serum was defined as 100 U/ml.

The cut offs (CO) of the ELISAs were chosen by receiver operating characteristic (ROC) curve analysis. We defined patients with CRPS as the fraction of true positives out of the positives (True positive rate, TPR) and healthy donors as the fraction of the false positives out of the negatives (False positive rate, FPR). Each ELISA was performed with 8 negative blood donors.

Using full recombinant protein of the inhibitor of growth protein 4 (p29ING4), the frequency of anti-IgG autoantibodies against p29ING4 was 15/46 (33%) in patients with CRPS, 14/46 (30%) in patients with SpA, 8/36 (22%) in patients with RA, 3/40 (8%) in patients with GPA, and 1/56(1%) in healthy donors.

The frequency of anti-IgA autoantibodies against p29ING4 was 18/46 (39%) in patients with CRPS, 12/46 (26%) in patients with SpA, 10/36 (28%) in patients with RA, 2/40 (3%) in patients with GPA, 4/56 (7%) in healthy donors. By combining both ELISAs, the frequency of autoantibodies against p29ING4 was 27/46 (59%) in patients with CRPS, 19/46 (40%) in patients with SpA, 14/36 (39%) in patients with RA, 3/40 (8%) in patients with GPA, and 4/56 (7%) in healthy donors.

By exclusion of patients with inflammatory back pain or polyarthritis, the specificity for CRPS within the groups of inflammatory joint diseases and febrile diseases increased to 100% and the sensitivity did not change.

By exclusion of patients with incidental trauma or inflammatory back pain and HLA-B27, the specificity for RA within the groups of inflammatory joint diseases and febrile diseases increased to 100% and the sensitivity did not change.

By exclusion of patients with polyarthritis and rheumatoid factors or anti-citrullinated antibodies, the specificity for RA within the groups of inflammatory joint diseases increased to 100% and the sensitivity decreased to 7/36 (19%) for IgG antibodies, 9/36 (25%) for IgA and 12/36 (33%) for IgA or IgG antibodies.

By using the detection of IgG antibodies against the peptide A1-23 of p29ING4, we could detect a similar percentage of patients as for the combined use of IgA and IgG antibodies against p29ING4. Since different patients with CRPS were analysed the sensitivity increased to 36/46 (78%) for CRPS. A similar effect could be observed for RA and SpA.

Aa1-23 peptide has a high similarity to ING1, 3 and 5, e.g peptide of p28ING5 MATAMYLEHYLDSIENLPCELQR (Seq. ID. No. 6), or, p27ING3 LYLEDYLEMIEQLPMDL (Seq. ID. No. 7).

We detected antibodies against ING5 in 1/5 patients with CRPS by protein array screening. Therefore, CRPS or other inflammatory joint diseases might target other ING proteins as well and using these proteins as antigens might increase the sensitivity for CRPS and other inflammatory joint diseases.

## Claims

1. A method for diagnosing the presence or the risk of development, or for the therapy control of the complex regional pain syndrome (CRPS) or other types of inflammatory diseases involving joints, preferably of CRPS I, in a subject comprising
analysing on a biological sample obtained from the subject for the presence of autoantibodies against an inhibitor of growth (ING) gene product, in particular, against p29ING4,
whereby the presence of autoantibodies against the ING gene product, in particular against p29ING4 is indicative of the presence or the risk of development, or for the therapy control of CRPS or other types of inflammatory diseases involving joints.

2. The method according to claim 1, **characterized in that** IgA and/or IgG autoantibodies are detected.

3. The method according to any one of the preceding claims, **characterized in that** the subject is a human and the autoantibodies are human autoantibodies.

4. The method according to any one of the preceding claims, **characterized in that** detection is performed using an immunoassay, preferably with direct or indirect coupling of one reactant to a detectable marker substance.

5. The method according to claim 4, **characterized in that** the detection is carried out using an ELISA, RIA, multiplex immunoassay or immunofluorescence assay, western blot, line assay, dot blot assay.

6. The method according to any one of the preceding claims wherein the biological sample is an isolated sample selected from blood, tissue or fluid preferably selected from hair, skin, nails, saliva, synovia, liquor and blood.

7. The method according to claim 6 wherein the biological sample is a body fluid, preferably blood, in particular, serum.

8. The method according to any one of the preceding claims being an in vitro method.

9. The method according to any one of the preceding claims allowing differentiation between CRPS and other types of diseases, in particular, arthrosis, or non-inflammatory diseases.

10. The method according to any one of the preceding claims for the stratification of the therapeutic regimen of a subject afflicted with CRPS or other types of inflammatory diseases involving joints, or being at risk of developing CRPS or other types of inflammatory diseases involving joints.

11. The method according to any one of the preceding claims for identifying the status of disease, in particular, the activity of the disease in a subject afflicted CRPS or other types of inflammatory diseases involving joints.

12. The use of a diagnostic kit in a method according to any one of claims 1 to 11 comprising means of determining autoantibodies against an ING gene product, in particular, against p29ING4 in a biological sample of a subject to be tested and instructions on how to use said test kit.

13. The use of a test kit according to claim 12 which is an ELISA, RIA, multiplex immunoassay, immunoblot or dot blot.

14. A use of an ING gene product, in particular, of p29ING4, or the immune reactive peptides thereof, in particular, a peptide including the amino acid sequence according to any one of Seq. ID. No. 1, 2, 3, 4, 5, 6, or 7 in the diagnosis, risk assessment or therapy control of CRPS or developing CRPS or other types of inflammatory diseases involving joints, in particular, in subjects undergoing surgery.

## Patentansprüche

1. Verfahren zur Diagnose des Vorhandenseins oder des Risikos einer Entwicklung oder für eine Therapiekontrolle des komplexen regionalen Schmerzsyndroms (CRPS) oder anderen Arten von Gelenke einschließenden Entzündungskrankheiten, bevorzugt von CRPS I, in einem Subjekt umfassend das Analysieren einer biologischen Probe erhalten von diesem Objekt auf das Vorhandensein von Autoantikörpern gegen ein Genprodukt des Wachstumsinhibitors (Inhibitor of Growth, ING), insbesondere gegen p29ING4, wobei das Vorhandensein der Autoantikörper gegen das ING Genprodukt insbesondere gegen p29ING4 indikativ ist für das Vorhandensein oder das Risiko einer Entwicklung, oder für die Therapiekontrolle von CRPS oder anderen Arten von Gelenke einschließenden Entzündungskrankheiten.

2. Verfahren nach Anspruch 1, **dadurch gekennzeichnet, dass** IgA und/oder IgG Autoantikörper detektiert werden.

3. Verfahren nach einem der vorherigen Ansprüche, **dadurch gekennzeichnet, dass** das Objekt ein Mensch ist und die Autoantikörper sind humane Antikörper.

4. Verfahren nach einem der vorherigen Ansprüche, **dadurch gekennzeichnet, dass** die Detektion durchgeführt wird mit einem Immunoassay, bevorzugt mit einem direkten oder indirekten Koppeln eines Reaktanten an eine nachweisbare Markersubstanz.

5. Verfahren nach Anspruch 4, **dadurch gekennzeichnet, dass** die Detektion durchgeführt wird mittels ELISA, RIA, Multiplex Immunoassay oder Immunoflureszenzassay, Western Blot, Line Assay, Dot Blot Assay.

6. Verfahren nach einem der vorherigen Ansprüche, wobei die biologische Probe eine isolierte Probe ist ausgewählt aus Blut, Gewebe oder Flüssigkeit bevorzugt ausgewählt aus Haar, Haut, Nägeln, Speichel, Synovialflüssigkeit, Liquor und Blut.

7. Verfahren nach Anspruch 6, wobei die biologische Probe eine Körperflüssigkeit, bevorzugt Blut, insbesondere Serum ist.

8. Verfahren nach einem der vorherigen Ansprüche, das ein *in vitro* Verfahren ist.

9. Verfahren nach einem der vorherigen Ansprüche, das die Differenzierung zwischen CRPS und anderen Arten von Erkrankungen, insbesondere Arthrose oder nicht-Entzündungserkrankungen ist.

10. Verfahren nach einem der vorherigen Ansprüche zur Stratifizierung des Therapieplans eines Objekts, das mit CRPS oder anderen Arten von Gelenke einschließenden Entzündungskrankheiten betroffen ist oder das ein Risiko der Entwicklung von CRPS oder anderen Arten von Gelenke einschließenden Entzündungskrankheiten besitzt.

11. Verfahren nach einem der vorherigen Ansprüche zur Identifikation des Status der Erkrankung insbesondere der Aktivität der Erkrankung in einem Objekt, das von CRPS oder anderen Arten von Gelenke einschließenden Entzündungskrankheiten betroffen ist.

12. Verwendung eines diagnostischen Kits in einem Verfahren nach einem der Ansprüche 1 bis 11 umfassend Mittel zum Bestimmen von Autoantikörpern gegen ein ING Genprodukt, insbesondere gegen p29ING4, in einer biologischen Probe eines zu testenden Objekts und Anweisungen zur Verwendung dieses Test Kits.

13. Verwendung eines Test Kits nach Anspruch 12, wobei dieses ein ELISA, RIA, Multiplex Immunoassay, Immunoblot oder Dot Blot ist.

14. Verwendung eines ING Genprodukts, insbesondere von p29ING4, oder den immunreaktiven Peptiden davon, insbesondere ein Peptid einschließlich der Amminosäuresequenz nach einem der SEQ ID Nrn. 1, 2, 3, 4, 5, 6 oder 7 in der Diagnose, Risikobewertung oder Therapiekontrolle von CRPS oder sich entwickelnder CRPS oder anderen Arten von Gelenke einschließenden Entzündungskrankheiten insbesondere in einem Objekt, das einem chirurgischen Eingriff unterworfen wird.

## Revendications

1. Méthode de diagnostic de la présence ou du risque de développement, ou de contrôle thérapeutique du syndrome douloureux régional complexe (SDRC) ou d'autres types de maladies inflammatoires impliquant les articulations, préférentiellement le SDRC I, chez un sujet, comprenant
l'analyse d'un échantillon biologique obtenu auprès du sujet en présence d'auto-anticorps contre un produit de gène inhibiteur de croissance (ING), en particulier contre p29ING4,
par laquelle la présence d'auto-anticorps contre le produit de gène ING, en particulier contre p29ING4, indique la présence ou le risque de développement, ou pour le contrôle thérapeutique du SDRC ou d'autres types de maladies inflammatoires impliquant les articulations.

2. Méthode selon la revendication 1, **caractérisée en ce que** des auto-anticorps IgA et/ou IgG sont détectés.

3. Méthode selon l'une quelconque des revendications précédentes, **caractérisée en ce que** le sujet est un humain et les auto-anticorps sont des auto-anticorps humains.

4. Méthode selon l'un quelconque des revendications précédentes, **caractérisée en ce que** la détection est mise en oeuvre par le biais d'un dosage immunologique, préférentiellement par couplage direct ou indirect d'un réactif avec une substance de type marqueur détectable.

5. Méthode selon la revendication 4, **caractérisée en ce que** la détection est mise en oeuvre en utilisant un dosage immunologique ELISA, RIA, multiplexé ou un dosage par immunofluorescence, un dosage Western Blot, un dosage en ligne, un dosage dot-blot.

6. Méthode selon l'une quelconque des revendications précédentes, où l'échantillon biologique est un échantillon isolé choisi parmi le sang, les tissus ou les fluides, préférentiellement choisi parmi les cheveux, la peau, les ongles, la salive, le liquide synovial et le sang.

7. Méthode selon la revendication 6, où l'échantillon biologique est un fluide corporel, préférentiellement le sang, en particulier le sérum.

8. Méthode selon l'une quelconque des revendications précédentes, qui est une méthode *in vitro.*

9. Méthode selon l'une quelconque des revendications précédentes permettant la différenciation entre le SDRC et d'autres types de maladies, en particulier l'arthrose ou les maladies non inflammatoires.

10. Méthode selon l'une quelconque des revendications précédentes pour la stratification du régime thérapeutique d'un sujet souffrant de SDRC ou d'autres types de maladies inflammatoires impliquant des articulations, ou présentant un risque de développer le SDRC ou d'autres types de maladies inflammatoires impliquant des articulations.

11. Méthode selon l'une quelconque des revendications précédentes pour l'identification du statut de maladies, en particulier l'activité de la maladie chez un sujet souffrant de SDRC ou d'autres types de maladies inflammatoires impliquant des articulations.

12. Utilisation d'un kit de diagnostic dans une méthode selon l'une quelconque des revendications 1 à 11 comprenant des moyens de détermination des auto-anticorps contre un produit de gène ING, en particulier contre p29ING4 dans un échantillon biologique d'un sujet à tester et des instructions sur l'utilisation dudit kit d'analyse.

13. Utilisation d'un kit d'analyse selon la revendication 12 qui est un dosage immunologique ELISA, RIA, multiplexé ou un dosage immunoblot ou dot-blot.

14. Utilisation d'un produit de gène ING, en particulier de p29ING4, ou des peptides immunoréactifs de celui-ci, en particulier un peptide incluant la séquence d'acides aminés selon l'une quelconque des Seq. ID. No. 1, 2, 3, 4, 5, 6 ou 7 dans le diagnostic, l'évaluation du risque ou le contrôle thérapeutique du SDRC ou de l'évolution du SDRC ou d'autres types de maladies inflammatoires impliquant des articulations, en particulier, chez les sujets soumis à une chirurgie.


### SEQUENCE LISTING

<110> Medizinische Hochschule Hannover
<120> Biomarker for inflammatory diseases involving joints, like CRPS
<130> 3096-032 EP
<160> 7
<170> PatentIn version 3.5
<210> 1
   <211> 23
   <212> PRT
   <213> homo sapiens
<400> 1
   Met Ala Ala Gly Met Tyr Leu Glu His Tyr Leu Asp Ser Ile Glu Asn
   1               5                   10                  15
   Leu Pro Phe Glu Leu Gln Arg
               20
<210> 2
   <211> 20
   <212> PRT
   <213> homo sapiens
<400> 2
   Phe Gln Leu Met Arg Asp Leu Asp Gln Arg Thr Glu Asp Leu Lys Ala
   1               5                   10                  15
   Glu Ile Asp Lys
               20
<210> 3
   <211> 29
   <212> PRT
   <213> homo sapiens
<400> 3
   Leu Ala Thr Glu Tyr Met Ser Ser Ala Arg Ser Leu Ser Ser Glu Glu
   1               5                   10                  15
   Lys Leu Ala Leu Leu Lys Gln Ile Gln Glu Ala Tyr Gly
               20                  25
<210> 4
   <211> 23
   <212> PRT
   <213> homo sapiens
<400> 4
   Ala Tyr Gly Lys Cys Lys Glu Phe Gly Asp Asp Lys Val Gln Leu Ala
   1               5                   10                  15
   Met Gln Thr Tyr Glu Met Val
               20
<210> 5
   <211> 21
   <212> PRT
   <213> homo sapiens
<400> 5
   Asp Lys His Ile Arg Arg Leu Asp Thr Asp Leu Ala Arg Phe Glu Ala
   1               5                   10                  15
   Asp Leu Lys Glu Lys
               20
<210> 6
   <211> 23
   <212> PRT
   <213> homo sapiens
<400> 6
   Met Ala Thr Ala Met Tyr Leu Glu His Tyr Leu Asp Ser Ile Glu Asn
   1               5                   10                  15
   Leu Pro Cys Glu Leu Gln Arg
               20
<210> 7
   <211> 17
   <212> PRT
   <213> homo sapiens
<400> 7
   Leu Tyr Leu Glu Asp Tyr Leu Glu Met Ile Glu Gln Leu Pro Met Asp
   1               5                   10                  15
   Leu